# EUROPÄISCHE PATENTSCHRIFT

(11) **EP 3 781 047 B1**
(45) Veröffentlichungstag und Bekanntmachung des Hinweises auf die Patenterteilung: **22.06.2022**
(21) Anmeldenummer: 19719259.4
(22) Anmeldetag: 18.04.2019
(51) Int. Cl.: A61B 17/128, A61B 17/28, A61B 17/00

(54) **CLIP-APPLIKATOR**
CLIP APPLICATOR
APPLICATEUR DE PINCES

(30) Priorität: 19.04.2018 DE 102018109427
(43) Veröffentlichungstag der Anmeldung: 24.02.2021
(73) Patentinhaber: Aesculap AG, 78532 Tuttlingen (DE)
(72) Erfinder: SAUTER, Wolfgang, 78603 Renquishausen (DE); PLEIL, Thomas, 78073 Bad Dürrheim (DE)
(74) Vertreter: Winter, Brandl - Partnerschaft mbB
(86) Internationale Anmeldenummer: PCT/EP2019/060199
(87) Internationale Veröffentlichungsnummer: WO 2019/202131

(56) Entgegenhaltungen:
- DE-A1- 10 137 915
- DE-U1- 29 800 876
- US-A- 3 777 760
- US-A- 6 066 159

## Beschreibung

Die vorliegende Erfindung betrifft ein chirurgisches Zangen-/Greifinstrument der Scheren- oder Pinzettenbauart und insbesondere einen Clip-Applikator zum Halten/Greifen/Anreichen eines Gewebe- vorzugsweise Aneurysmen-Clips.

### Hintergrund der Erfindung

Zum Anreichen oder Übergeben eines Gewebe-/Aneurysmen-Clips möchten Chirurgen am hierfür verwendeten Clip-Applikator eine Sperre, die sicherstellt, dass der Clip während der Übergabe nicht aus dem Applikator fällt, selbst dann, wenn die Halte/Betätigungsgriffe des Clip-Applikators kurzfristig losgelassen werden. Eine solche Sperre soll zum einen ein Verlieren/Abfallen des Clips verhindern, darf aber zum anderen während des Operationsvorgangs den Chirurgen nicht irritieren oder übergebührlich behindern.

### Stand der Technik

Aus dem Stand der Technik sind allgemein chirurgische Halte-/Greifinstrumente der Scheren- oder Pinzettenbauart bekannt, welche mit sogenannten Durchlaufsperren ausgerüstet sind, die in der Regel am proximalen Ende (Betätigungsabschnitt) des Halte-/Greifinstruments angeordnet sind und aus einer Kulissenbahn auf Seiten der einen Branche und einer Umlaufnocke auf Seiten der anderen Branche besteht, die beweglich an der anderen Branche gelagert ist. Werden die beiden Branchen im Bereich des Betätigungsabschnitts des Instruments zusammengedrückt, klinkt sich die Umlaufnocke in die Kulissenbahn an deren Eingang ein, die eine Hinterschneidung in Form einer konkaven Krümmung ausgebildet ist, in welche sich die Umlaufnocke beim erstmaligen Freigeben/Loslassen der beiden Branchen verrastet und so das Instrument in einem geschlossenen Zustand hält. Werden darauf hin die beiden Branchen erneut (zweitmalig) betätigt, wandert die Umlaufnocke längs der Kulissenbahn aus der konkav gekrümmten Hinterschneidung in Richtung Ausgang der Kulissenbahn fort und gibt schließlich die beiden Branchen frei.

Eine andere Konstruktionsvariante sieht Bleche vor, die in einer exponierten Lage an jeder der Branchen montiert sind und ab einer ersten Winkelstellung X der Branchen aufeinander auffahren, bei einer Winkelstellung von beispielsweise X-5° sich gegenseitig hintergreifen und so eingerastet werden und bei einer Winkelstellung von beispielsweise X-10° überfahren sind.

Ein Nachteil dieser Lösung besteht darin, dass der ganze Winkelbereich X-10° auch beim Appliziervorgang des Clips überfahren werden muss. Das bei diesem Überfahren entstehende Rückfedern der Sperre wird als unangenehm empfunden und sorgt für Irritationen beim Appliziervorgang.

DE 101 37 915 A1 offenbart ein zangen- oder pinzettenförmiges chirurgisches Instrument mit zwei gegeneinander schwenkbaren Branchen, mit einem Federarm an einer Branche und einem Halteglied an der anderen Branche, an welcher der Federarm beim Annähern der Branchen derart lösbar festlegbar ist, daß die Branchen in einer angenäherten Verriegelungsstellung verbleiben.

US 6,066,159 offenbart ein chirurgisches Instrument, bestehend aus zwei mittels eines Gelenks schwenkbar miteinander verbundenen Branchen, die jeweils mit einem Handgriff und Arretiermitteln ausgestattet sind, mittels derer die Branchen miteinander arretierbar sind, wobei die Handgriffe durch Kopplungsmittel lösbar mit der jeweiligen Branche verbunden sind.

US 3,777,760 offenbart ein chirurgisches Instrument das in einem Stück aus einem elastischen Material gebildet ist und ein paar langgestreckte Klemmelemente aufweist, die sich ausgehend von einem Greifabschnitt erstrecken, zum Einführen von absorbierendem Material gespreizt und zum sicheren Halten des absorbierenden Materials miteinander verriegelt werden können.

### Kurzbeschreibung der Erfindung

Angesichts vorstehender Problematik ist es die Aufgabe der vorliegenden Erfindung, ein chirurgisches Greif-/Halteinstrument vorzugsweise der Scheren-/Zangenbauart oder Pinzettenbaurart mit einer Sperre/Umlaufsperre bereitzustellen, bei welchem Irritationen durch die Sperre verringert oder vermieden werden.

Diese Aufgabe wird durch ein entsprechendes chirurgisches Instrument mit den Merkmalen des Patentanspruchs 1 gelöst. Vorteilhafte Ausgestaltungen der Erfindung sind Gegenstand der Unteransprüche.

Der Kern der vorliegenden Erfindung besteht demzufolge darin, die (Umlauf-) Sperre in einem Bereich zwischen dem Greif-/Halteabschnitt (distaler Effektorabschnitt des Instruments) und dem (proximalen, manuellen) Betätigungsabschnitt des chirurgischen Instruments anzuordnen und die Instrumentenbranchen in diesem Zwischenbereich so zu gestalten, dass bei einer (manuellen) Betätigung des Instruments ausschließlich am hierfür vorgesehenen Betätigungsabschnitt des Instruments, die Sperre nicht in Sperrzustand bringbar ist. Dieser Sperrzustand ist erfindungsgemäß nur erreichbar, indem das Instrument vorzugsweise zusätzlich zur Betätigung am hierfür vorgesehenen Betätigungsabschnitt im (außerhalb des Betätigungsabschnitts befindlichen) Zwischenbereich (welcher den Greif-/Halteabschnitt mit dem Betätigungsabschnitt verbindet) betätigt wird. Genauer gesagt ist vorgesehen, dass die Zwischenbereiche die ausschließlichen Betätigungselemente zum Ein- und Ausrücken der Instrumentensperre darstellen.

Klarstellungshalber sei an dieser Stelle erwähnt, dass es sich bei den (manuellen) Betätigungsabschnitten der Instrumentenbranchen um die Instrumentenbetätigungsabschnitte handelt, mit denen die Instrumentenbranchen, beispielsweise durch den Benutzer, relativ zueinander verschwenkt werden können, also das Instrument an diesen Instrumentenbetätigungsabschnitten geöffnet und geschlossen werden kann bzw. in seiner Öffnungs- und Schließrichtung betätigt werden kann. Anders ausgedrückt ist die Instrumentenbetätigung im Sinne von Öffnen und Schließen des Instruments zu verstehen. Dabei wird die Instrumentenbetätigung mittels der (proximalen) Instrumentenbetätigungsabschnitte vorgenommen. Bei der Instrumentensperre handelt es sich um eine wieder lösbare Instrumentensperre.

Das erfindungsgemäße chirurgische Greif-/Halteinstrument hat demnach zwei relativ zueinander bewegliche Instrumentenbranchen, die nach Art einer Schere/Zange oder nach Art einer Pinzette schwenkbar miteinander gekoppelt sind. Diese beiden Branchen bilden an ihrem jeweils distalen Endabschnitt den Halte-/Greifabschnitt und an ihrem jeweils proximalen Endabschnitt den vorzugsweise manuellen Betätigungsabschnitt beispielsweise einen Instrumentengriff für ein Öffnen und Schließen des Instruments am Greif-/Halteabschnitt der Branchen (nachfolgend als Instrumentenbetätigungsabschnitt bezeichnet) aus. Der Halte-/Greifabschnitt und der Betätigungsabschnitt jeder Branche ist durch den Zwischenbereich miteinander vorzugsweise einstückig verbunden, in welchen sich beispielsweise im Fall einer Scheren-/Zangenbauweise ein Scharniergelenk befindet, über welches die beiden Branchen miteinander schwenkbar gekoppelt sind. In dem Zwischenbereich, im Fall der Scheren-/Zangenbauart vorzugsweise bezüglich des Scharniergelenks auf Seiten der Betätigungsabschnitte, ist die (Branchen-)Sperre, d.h. die miteinander in Sperr/Rasteingriff zu bringenden Sperrelemente vorzugsweise bekannter Bauart (Nocken, Kulissenbahnen, Bleche, etc.) angeordnet.

In diesem Fall sind die Branchen in deren jeweiligen Zwischenbereichen (außerhalb der jeweiligen Betätigungsabschnitte für ein Öffnen und Schließen des Instruments) erfindungsgemäß so geformt, vorzugsweise bogenförmig, dass die sich gegenüberliegenden Sperrelemente der Instrumentensperre außer Eingriff bleiben, wenn das Instrument lediglich an den Betätigungsabschnitten (Instrumentengriff) der Branchen (vorschriftsmäßig) für ein Greifen/Halten eines Gegenstands/Gewebes insbesondere eines Aneurysmen-Clips (Öffnen und Schließen) vollständig betätigt ist (d.h. bis zur/zum maximal möglichen/zulässigen Betätigungsstellung/Betätigungszustand, insbesondere Schließzustand / Schließposition). Das erfindungsgemäße Greif-/Halteinstrument bildet demzufolge einen zweiten Betätigungsabschnitt / Betätigungselement (ausschließlich) zum Sperren/Entsperren bzw. Einrücken / Ausrücken der Instrumentensperre (nachfolgend als Sperrbetätigungsabschnitt bezeichnet) aus, bei dessen erstmaliger (manueller) Betätigung bei bereits betätigtem Instrumentenbetätigungsabschnitt die Sperre in Sperrzustand bringbar ist, ohne dass hierbei am Betätigungszustand des Instrumentenbetätigungsabschnitt weitere Manipulationen vorgenommenen werden müssen. Soll das Instrument wieder geöffnet werden, muss zunächst der Sperrbetätigungsabschnitt (ohne weitere Manipulationen am Instrumentenbetätigungsabschnitt) zweitmalig betätigt werden, um so nach bekannter Art die Sperre (Umlaufsperre) zu lösen und anschließend der Instrumentenbetätigungsabschnitt freigegeben werden, um das Instrument zu öffnen.

Funktional lässt sich die Erfindung demnach so beschreiben, dass die Betätigung des Instruments für ein Öffnen und Schließen funktional von der Betätigung des Instruments für ein Sperren und Entsperren des Instruments (ausschließlich durch die erfindungsgemäße Positionierung der Sperre im Zwischenbereich) voneinander getrennt sind und somit individuell (wahlweises Zuschalten der Sperre) durchgeführt werden können.

Insbesondere erfolgt das Ein- und Ausrücken der Instrumentensperre durch elastische Deformation der Zwischenbereiche, insbesondere ausschließlich durch elastische Deformation der Zwischenbereiche.

Vorzugsweise bilden die beiden Sperrelemente eine Umlaufsperre. Besonders bevorzugt bewegen sich die beiden Sperrelemente mittels einer erstmaligen Betätigung der Instrumentensperre aufeinander zu, um miteinander zu verrasten, und bewegen sich mittels einer zweitmaligen Betätigung der Instrumentensperre erneut aufeinander zu, um sich wieder voneinander zu lösen.

Bevorzugt bilden die beiden Instrumentenbranchen zwischen deren jeweiligen Zwischenbereichen (bzw. Betätigungselementen zum Ein- und Ausrücken der Instrumentensperre) einen Spalt aus, wenn das Instrument lediglich an den proximalen Betätigungsabschnitten der Instrumentenbranchen bis zu einer maximal möglichen Betätigungsposition, insbesondere (einer maximal möglichen) Schließposition / Schließzustand, für ein Greifen/Halten eines Gegenstands/Gewebes betätigt ist. Durch erstmaliges Betätigen der Instrumentensperre durch Druckbeaufschlagung der beiden Zwischenbereiche derart, dass diese sich aufeinander zu bewegen, schließt sich der Spalt und die beiden sich gegenüberliegenden Sperrelemente verrasten miteinander. Insbesondere durch zweitmaliges Betätigen der Instrumentensperre durch Druckbeaufschlagung der beiden Zwischenbereiche derart, dass diese sich aufeinander zu bewegen, lösen sich die beiden Sperrelemente aus dem Rasteingriff wieder.

Besonders bevorzugt sind die Sperrelemente in den Zwischenbereichen der Instrumentenbranchen jeweils so angeordnet, dass diese durch eine Seitwärtsbewegung senkrecht zur Öffnungs-/Schließrichtung der Instrumentenbranchen, bevorzugt senkrecht zur Schwenkebene der Instrumentenbranchen, oder durch eine Bewegung in der Öffnungs-/Schließrichtung der Instrumentenbranchen, bevorzugt in der Schwenkebene der Instrumentenbranchen, miteinander verrasten und insbesondere wieder freigegeben werden können.

### Kurzbeschreibung der Figuren

Die Erfindung wird nachstehend anhand eines bevorzugten Ausführungsbeispiels unter Bezugnahme auf die begleitenden Figuren näher erläutert. Es zeigen:
Fig. 1 ein chirurgisches Halte-/Greifinstrument vorzugsweise der Zangenbauart insbesondere ein Aneurysmenclip-Applikator gemäß einem bevorzugten Ausführungsbeispiel der Erfindung in einem unbetätigten/geöffneten Zustand

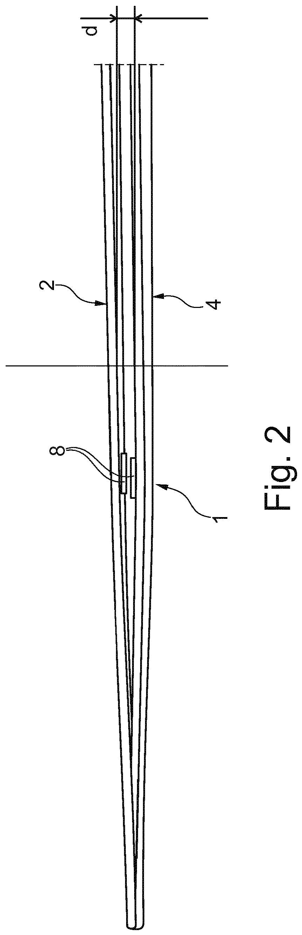

Fig. 2 das chirurgische Halte-/Greifinstrument gemäß Fig. 1 in einem betätigten/geschlossenen Zustand bei unbetätigter/entsperrter Instrumentensperre und

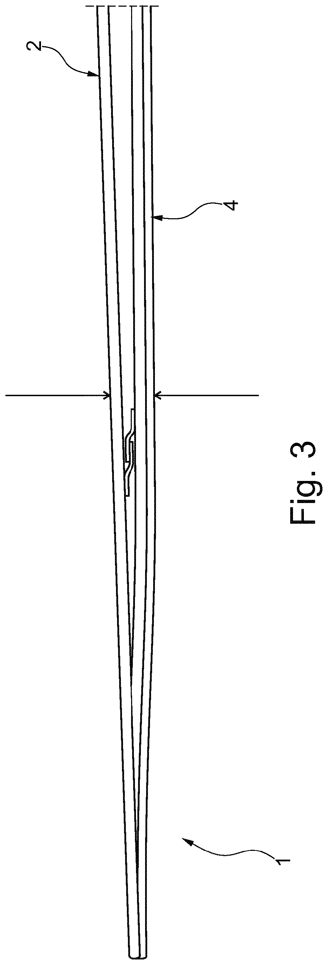

Fig. 3 das chirurgische Halte-/Greifinstrument gemäß Fig. 2 in einem betätigten/geschlossenen Zustand bei betätigter/gesperrter (zugeschalteter) Instrumentensperre.

### Figurenbeschreibung

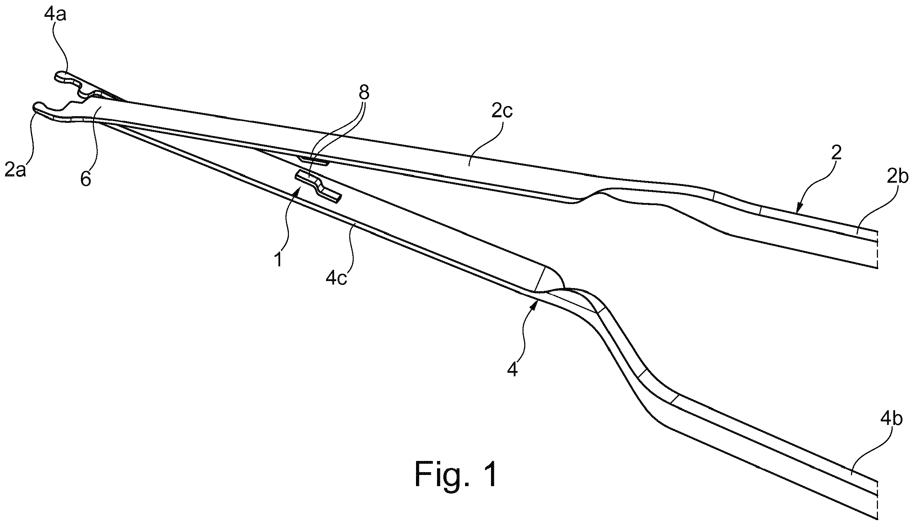

Gemäß der Fig. 1 ist das chirurgische Greif-/Halteinstrument 1 des bevorzugten Ausführungsbeispiels der vorliegenden Erfindung ein Aneurysmenclip-Applikator der Zangen-/Scherenbauart mit zwei vorzugsweise balkenförmigen Instrumentenbranchen 2, 4, die jeweils einen distalen Halte-/Greifabschnitt 2a, 4a, einen proximalen Instrumenten-Betätigungsabschnitt 2b, 4b und einen den distalen Halte-/Greifabschnitt 2a, 4a sowie die proximalen Instrumenten-Betätigungsabschnitt 2b, 4b stoffeinstückig koppelnden/verbindenden Zwischenbereich 2c, 4c bilden. Im vorliegenden Ausführungsbeispiel sind die beiden Instrumentenbranchen 2, 4 über ein Schwenkscharnier vorzugsweise in Form eines Scharnierstifts oder Bolzens 6 miteinander schwenkgekoppelt, der im Zwischenbereich 2c, 4c, vorzugsweise am distalen Ende des Zwischenbereichs 2c, 4c angeordnet ist und hierfür in jeweils eine Durchgangsbohrung (nicht näher gezeigt) an den Instrumentenbranchen 2, 4 eingesetzt ist.

An dieser Stelle sei darauf hingewiesen, dass das chirurgische Greif-/Halteinstrument auch nach Art einer Pinzette ausgebildet sein kann mit ebenfalls zwei Instrumentenbranchen, die jeweils einen distalen Halte-/Greifabschnitt, einen proximalen Instrumenten-Betätigungsabschnitt und einen den distalen Halte-/Greifabschnitt sowie die proximalen Instrumenten-Betätigungsabschnitt stoffeinstückig koppelnden Zwischenbereich definieren, wobei in diesem Fall jedoch die beiden Instrumentenbranchen über ein Federscharnier (Blattfederabschnitt) am jeweils proximalen Ende der Instrumenten-Betätigungsabschnitte miteinander gekoppelt sind.

Wie aus der Fig. 1 gut erkennbar ist, kann der Halte-/Greifabschnitt 2a, 4a jeder Instrumentenbranche 2, 4 dafür ausgebildet und vorbereitet sein, im gegenseitigen Zusammenwirken ein Implantat, beispielsweise ein Aneurysmenclip zu greifen und zu halten. Es ist aber auch denkbar, die erfindungsgemäßen Halte-/Greifabschnitte 2a, 4a für das Greifen und Halten eines anderen chirurgischen Gegenstands, z.B. eine Nadel oder dergleichen oder von Patientengewebe zu optimieren.

Die proximalen Instrumenten-Betätigungsabschnitte 2b, 4b sind dafür vorgesehen und angepasst, manuell, d.h. mit der Hand ergriffen zu werden, um diese für ein Schließen des Instruments 1 (der distalen Greif-/Halteabschnitte 2a, 4a) gegeneinander (aufeinander zu) zu bewegen bzw. zu verschwenken. Dabei ist für ein (maximales) Öffnen und (maximales) Schließen des erfindungsgemäßen chirurgischen Instruments 1 ein vorbestimmter (maximaler) Bewegungsweg bzw. Schwenkwinkel der Branchen 2, 4/proximalen Instrumenten-Betätigungsabschnitte 2b, 4b notwendig bzw. vorgesehen, der beispielsweise durch Anschläge in Öffnung- und/oder Schließrichtung begrenzt sein kann (nicht weiter dargestellt).

Schließlich ist das chirurgische Instrument 1 mit einer Instrumentensperre 8 ausgerüstet, die erfindungsgemäß im Zwischenbereich 2c, 4c jeder Instrumentenbranche 2, 4, d.h. außerhalb der Instrumentenbetätigungsabschnitte 2b, 4b und außerhalb der Greif-/Halteabschnitte 2a, 4a jeder Instrumentenbranche 2, 4 positioniert ist. Vorzugsweise ist die Instrumentensperre 8 im Zwischenbereich 2c, 4c zwischen den proximalen Betätigungsabschnitten 2b, 4b und dem Scharnier(-stift) 6 angeordnet.

Die Instrumentensperre 8 ist vorliegend nach bekannter Bauart aufgebaut, d.h. sie besteht im Wesentlichen aus zwei miteinander verrastbaren Sperrenelementen beispielsweise in Form von Hinterschneidungen ausbildenden Blechen, Rastnocken und/oder Kulissenbahnen, die in sich gegenüberliegender sowie einander zugewandter Weise an den Instrumentenbranchen 2, 4 angeordnet sind, derart, dass bei einer erstmaligen Betätigung der Sperre 8 durch aufeinander zu bewegen der beiden Sperrenelemente diese miteinander verrasten und so die Instrumentenbranchen 2, 4 bzw. das Instrument 1 in geschlossenem Zustand sperren und bei einer zweitmaligen Betätigung der Sperre 8 (z.B. ebenfalls durch aufeinander zu bewegen der beiden Sperrenelemente) sich diese voneinander lösen und so die Instrumentenbranchen 2, 4 bzw. das Instrument 1 für dessen Öffnen wieder freigeben.

An dieser Stelle sei darauf hingewiesen, dass die Instrumentensperre 8 jedwede Ausgestaltung gemäß dem Stand der Technik aufweisen kann. Beispielsweise kann an einer der Branchen 2, 4 eine Führungskulisse angeordnet und an der gegenüberliegenden Branche 4, 2 eine Umlaufnocke angelenkt sein, wodurch sich eine Instrumentensperre 8 der Umlaufbauform ergibt. Es ist aber auch möglich, jede Instrumentenbranche 2, 4 mit einem vorzugsweise hakenförmig gestalteten Blech auszustatten, die so angeordnet sind, dass diese durch eine Seitwärtsbewegung (senkrecht zur Öffnung-/Schließrichtung) der Instrumentenbranchen 2, 4 miteinander verrastet und wieder freigegeben werden können (lateral versetzte Sperre).

Erfindungsgemäß ist der Zwischenbereich 2c, 4c wenigstens einer Branche 2, 4 sowie zumindest abschnittsweise so geformt, dass die Instrumentensperre 8 bzw. deren Sperrenelemente nicht verrastet werden kann/können, wenn das chirurgische Instrument 1 ausschließlich an dessen proximalen Instrumentenbetätigungsabschnitten 2b, 4b bis zu der maximalen Betätigungsposition betätigt wird. Im Konkreten sind die Zwischenbereiche 2c, 4c zumindest einer, vorzugsweise beider Instrumentenbranchen 2, 4 beispielsweise bogenförmig gestaltet, derart, dass bei Betätigung ausschließlich der proximalen Betätigungsabschnitte 2b, 4b bis zu deren maximaler Betätigungs-/Schließposition sich zwischen den Instrumentenbranchen 2, 4 zumindest an den Zwischenbereichen 2c, 4c ein Spalt d ergibt/verbleibt, der so bemessen ist, dass die beiden einander zugewandten Sperrenelemente der Instrumentensperre 8 nicht in Rasteingriff kommen/bringbar sind.

Erfindungsgemäß bildet der Zwischenbereich 2c, 4c zumindest der einen, vorzugsweise jeder Instrumentenbranche 2, 4 einen zum proximalen Instrumentenbetätigungsabschnitt 2b, 4b zusätzlichen Sperrenbetätigungsabschnitt, der getrennt vom proximalen Instrumentenbetätigungsabschnitt 2b, 4b (ausschließlich) zum wahlweisen Sperren und Freigeben der Instrumentensperre 8 (bei bereits geschlossenem Instrument/bei maximaler Betätigungs-/Schließposition der proximalen Instrumentenbetätigungsabschnitte 2b, 4b) betätigbar ist.

Demzufolge kann der Zwischenbereich 2c, 4c zumindest einer/jeder Instrumentenbranche 2, 4 an deren einer Seite eine Betätigungsfläche/Betätigungsstruktur aufweisen, die auch als Positionsmarkierung für die Druck-/Kraftbeaufschlagung unmittelbar des Zwischenbereichs 2c, 4c dient und ggf. ergonomisch beispielsweise an einen Finger/Daumen angepasst sein kann. D.h., dass das erfindungsgemäße chirurgische Greif-/Halteinstrument 1 bzw. dessen Instrumentenbranchen 2, 4 jeweils zwei Betätigungsabschnitte hat/haben, nämlich den proximalen Instrumentenbetätigungsabschnitt 2b, 2c ausschließlich für ein Schließen (und Öffnen) des Instruments 1 sowie den im Zwischenbereich 2c, 4c angeordneten/durch diesen ausgebildeten Sperrenbetätigungsabschnitt ausschließlich für ein Sperren und/oder Freigeben der Instrumentensperre 8.

Die Funktionsweise des erfindungsgemäßen chirurgischen Greif-/Halteinstruments lässt sich wie folgt zusammenfassen:
Zunächst lässt sich das erfindungsgemäße Instrument 1 an den proximalen Instrumentenbetätigungsabschnitten 2b, 4b manuell wie ein aus dem Stand der Technik bekanntes Instrument dieser Gattung schließen, indem die relativ schwenkbar gekoppelten Branchen 2, 4 an deren proximalen Instrumentenbetätigungsabschnitten 2b, 4b aufeinander zu geschwenkt werden, bis ggf. eine maximale Schließposition erreicht ist. Da bei dieser Instrumentenbetätigung die Sperrenelemente der Instrumentensperrre 8 nicht in Rasteingriff kommen, da diese Infolge der erfindungsgemäßen Formgebung der Zwischenbereiche 2c, 4c sowie der besonderen Positionierung der Sperre 8 im Zwischenbereich 2c, 4c der Branchen 2, 4 einen Abstand zueinander beibehalten, kann das Instrument 1 einfach wieder geöffnet werden, indem die Betätigungsabschnitte 2b, 4b losgelassen werden.

Um das Instrument 1 in Schließposition wahlweise zu sperren, muss die Sperre durch (zusätzliches) Betätigen des Sperrenbetätigungsabschnitts/Zwischenbereichs 2c, 4c zugeschaltet werden. D.h. nach Betätigen der proximalen Instrumentenbetätigungsabschnitte 2b, 4b für ein Schließen des Instruments 1 wird zusätzlich die Sperre 8 betätigt, indem die Zwischenbereiche 2c, 4c durch direkt darauf einwirkende Druckbeaufschlagung aufeinander zu bewegt werden, um den bei der Betätigung ausschließlich der proximalen Instrumentenbetätigungsabschnitte 2b, 4b dazwischen verbliebenden Spalt d zu schließen und so die beiden sich gegenüberliegenden Sperrenelemente zu verrasten.

Soll das so verrastete Instrument wieder geöffnet werden, müssen die Zwischenbereiche 2c, 4c erneut direkt aufeinander zubewegt werden (bei ggf. maximaler Schließposition der proximalen Instrumentenbetätigungsabschnitte 2b, 4b), um die beiden Sperrenelemente außer Rasteingriff zu bringen, worauf die proximalen Instrumentenbetätigungsabschnitte 2b, 4b losgelassen werden können, um das Instrument 1 jetzt zu öffnen.

Hieraus ergeben sich gegenüber den bekannten Instrumenten dieser Gattung die folgenden Vorteile:
- Keine haptische Irritation des Chirurgen beim Appliziervorgang (Öffnen und Schließen);
- Erleichtertes gezieltes Einrasten der Instrumentensperre;
- Weniger störanfällig (kein unbeabsichtigtes Entsperren bei zufälligem Betätigen der proximalen Instrumenten-Betätigungsabschnitte, keine Schaltfehler durch verbogene Sperrenelemente);
- Vereinfachte und sichere Reinigbarkeit;
- Vereinfachte Herstellung (keine Montage/Einstellung);
- Reduzierte Herstellungskosten;
- Flachere Lernkurve als wie mit Instrumenten bekannter Bauart;
- Manipulationssicher;
- Wahlweise Zuschaltbarkeit der Sperre ist gegeben.

## Patentansprüche

1. Chirurgisches Greif-/Halteinstrument mit zwei relativ verschwenkbar gekoppelten Instrumentenbranchen (2, 4), die jeweils einen distalen Greif-/Halteabschnitt (2a, 4a), einen proximalen Instrumentenbetätigungsabschnitt (2b, 4b) und einen die distalen und proximalen Abschnitte vorzugsweise stoffeinstückig verbindenden Zwischenbereich (2c, 4c) haben, und mit einer Instrumentensperre (8), deren miteinander in einen Rasteingriff bringbare Sperrelemente an den Instrumentenbranchen (2, 4) angeordnet oder ausgebildet sind, wobei die Betätigung des Instruments (1) für ein Öffnen und Schließen und die Betätigung des Instruments (1) für ein Sperren und Entsperren des Instruments (1) durch die Positionierung der Instrumentensperre (8) im Zwischenbereich (2c, 4c) der Instrumentenbranchen (2, 4) funktional voneinander getrennt sind und somit individuell durchgeführt werden können, **dadurch gekennzeichnet, dass** die Zwischenbereiche (2c, 4c) der Instrumentenbranchen (2, 4) die ausschließlichen Betätigungselemente für ein Ein- und Ausrücken der Instrumentensperre (8) bilden.

2. Chirurgisches Greif-/Halteinstrument nach Anspruch 1, **dadurch gekennzeichnet, dass** die Instrumentenbranchen (2, 4) balkenförmig sind.

3. Chirurgisches Greif-/Halteinstrument nach Anspruch 2, **dadurch gekennzeichnet, dass** der Rast-/Sperrzustand der Instrumentensperre (8) nur erreichbar ist, indem das Instrument (1) zusätzlich zur Betätigung unmittelbar der proximalen Betätigungsabschnitte (2b, 4b) in den außerhalb der Betätigungsabschnitte (2b, 4b) befindlichen Zwischenbereichen (2c, 4c), welche die distalen Greif-/Halteabschnitte (2a, 4a) mit den Betätigungsabschnitten (2b, 4b) verbinden, betätigt wird.

4. Chirurgisches Greif-/Halteinstrument nach Anspruch 3, **dadurch gekennzeichnet, dass** die zwei relativ zueinander beweglichen Instrumentenbranchen (2, 4) nach Art einer Schere/Zange oder nach Art einer Pinzette schwenkbar miteinander gekoppelt sind und die Betätigungsabschnitte (2b, 4b) jeweils vorzugsweise manuell kraftbeaufschlagbar sind und weitervorzugsweise einen Instrumentengriff für ein Öffnen und Schließen des Instruments (1) ausbilden, wobei im Fall einer Scheren-/Zangenbauweise ein Scharniergelenk (6) vorgesehen ist, über welches die beiden Instrumentenbranchen (2, 4) miteinander schwenkbar gekoppelt sind, wobei die Instrumentensperre (8) in dem Zwischenbereich (2c, 4c) bezüglich des Scharniergelenks (6) auf Seiten der Betätigungsabschnitte (2b, 4b), angeordnet ist.

5. Chirurgisches Greif-/Halteinstrument nach einem der vorstehenden Ansprüche, **dadurch gekennzeichnet, dass** die Instrumentenbranchen (2, 4) in deren jeweiligen Zwischenbereichen (2c, 4c) außerhalb der proximalen Betätigungsabschnitte (2b, 4b) für ein Öffnen und Schließen des Instruments (1) so geformt sind, vorzugsweise bogenförmig, dass die sich gegenüberliegenden Sperrelemente der Instrumentensperre (8) außer Eingriff bleiben, wenn das Instrument (1) lediglich an den proximalen Betätigungsabschnitten (2b, 4b) der Instrumentenbranchen (2, 4) bis zu einer maximal möglichen Betätigungsposition, insbesondere Schließposition, für ein Greifen/Halten eines Gegenstands/Gewebes, insbesondere eines Aneurysmen-Clips, betätigt ist.

6. Chirurgisches Greif-/Halteinstrument nach einem der vorstehenden Ansprüche, **dadurch gekennzeichnet, dass** das Ein- und Ausrücken der Instrumentensperre (8) durch elastische Deformation der Zwischenbereiche (2c, 4c) erfolgt, insbesondere das Ein- und Ausrücken der Instrumentensperre (8) ausschließlich durch elastische Deformation der Zwischenbereiche (2c, 4c) erfolgt.

7. Chirurgisches Greif-/Halteinstrument nach einem der vorstehenden Ansprüche, **dadurch gekennzeichnet, dass** die beiden Sperrelemente eine Umlaufsperre bilden, insbesondere sich mittels einer erstmaligen Betätigung der Instrumentensperre (8) aufeinander zu bewegen, um miteinander zu verrasten, und die beiden Sperrelemente sich mittels einer zweitmaligen Betätigung der Instrumentensperre (8) erneut aufeinander zu bewegen, um sich wieder voneinander zu lösen.

8. Chirurgisches Greif-/Halteinstrument nach einem der vorstehenden Ansprüche, **dadurch gekennzeichnet, dass** die beiden Instrumentenbranchen (2, 4) zwischen deren jeweiligen Zwischenbereichen (2c, 4c) einen Spalt (d) bilden, wenn das Instrument (1) lediglich an den proximalen Betätigungsabschnitten (2b, 4b) der Instrumentenbranchen (2, 4) bis zu einer maximal möglichen Betätigungsposition, insbesondere Schließposition, für ein Greifen/Halten eines Gegenstands/Gewebes betätigt ist, und durch erstmaliges Betätigen der Instrumentensperre (8) durch Druckbeaufschlagung der beiden Zwischenbereiche (2c, 4c) derart, dass diese sich aufeinander zu bewegen, der Spalt (d) sich schließt und die beiden sich gegenüberliegenden Sperrelemente miteinander verrasten und insbesondere durch zweitmaliges Betätigen der Instrumentensperre (8) durch Druckbeaufschlagung der beiden Zwischenbereiche (2c, 4c) derart, dass diese sich aufeinander zu bewegen, die beiden Sperrelemente sich aus dem Rasteingriff wieder lösen.

9. Chirurgisches Greif-/Halteinstrument nach einem der vorstehenden Ansprüche, **dadurch gekennzeichnet, dass** die Sperrelemente in den Zwischenbereichen (2c, 4c) der Instrumentenbranchen (2, 4) jeweils so angeordnet sind, dass diese durch eine Seitwärtsbewegung senkrecht zur Öffnungs-/Schließrichtung der Instrumentenbranchen (2, 4) oder durch eine Bewegung in der Öffnungs-/Schließrichtung der Instrumentenbranchen (2, 4) miteinander verrasten und insbesondere wieder freigegeben werden können.

## Claims

1. Surgical gripping/holding instrument having two instrument branches (2, 4) which are coupled pivotably with respect to each other and which each have a distal gripping/holding portion (2a, 4a), a proximal instrument actuating portion (2b, 4b) and an intermediate region (2c, 4c) which connects the distal and proximal portions preferably integrally, and having an instrument lock (8) whose locking elements which can be brought into a latching engagement with each other are arranged or formed on the instrument branches (2, 4), wherein the actuation of the instrument (1) for opening and closing and the actuation of the instrument (1) for locking and unlocking the instrument (1) are functionally separated by the positioning of the instrument lock (8) in the intermediate region (2c, 4c) of the instrument branches (2, 4) and thus can be carried out individually, **characterized in that** the intermediate regions (2c, 4c) of the instrument branches (2, 4) form the exclusive actuating elements for engaging and disengaging the instrument lock (8).

2. Surgical gripping/holding instrument according to claim 1, **characterized in that** the instrument branches (2, 4) are bar shaped.

3. Surgical gripping/holding instrument according to claim 2, **characterized in that** the latching/locking state of the instrument lock (8) can only be achieved by actuating the instrument (1) in addition to directly actuating the proximal actuating portions (2b, 4b), in the intermediate regions (2c, 4c) located outside the actuating portions (2b, 4b) and connecting the distal gripping/holding portions (2a, 4a) to the actuating portions (2b, 4b).

4. Surgical gripping/holding instrument according to claim 3, **characterized in that** the two instrument branches (2, 4) which are movable relative to each other are pivotably coupled to each other in the manner of scissors/forceps or in the manner of tweezers, and the actuating portions (2b, 4b) can be applied with preferably manual force and more preferably form an instrument handle for opening and closing the instrument (1), wherein in the case of a scissors/forceps design, a hinge joint (6) is provided, via which the two instrument branches (2, 4) are pivotably coupled to each other, wherein the instrument lock (8) is arranged in the intermediate region (2c, 4c) on the side of the actuating portions (2b, 4b) relative to the hinge joint (6).

5. Surgical gripping/holding instrument according to one of the preceding claims, **characterized in that** the instrument branches (2, 4) in their respective intermediate regions (2c, 4c) outside the proximal actuating portions (2b, 4b) are shaped, preferably arcuately, for opening and closing the instrument (1), such that the opposite locking elements of the instrument lock (8) remain disengaged when the instrument (1) is actuated only at the proximal actuating portions (2b, 4b) of the instrument branches (2, 4) up to a maximum possible actuating position, in particular closed position, for gripping/holding an object/tissue, in particular an aneurysm clip.

6. Surgical gripping/holding instrument according to one of the preceding claims, **characterized in that** the engagement and disengagement of the instrument lock (8) is effected by elastic deformation of the intermediate regions (2c, 4c), in particular the engagement and disengagement of the instrument lock (8) is effected exclusively by elastic deformation of the intermediate regions (2c, 4c).

7. Surgical gripping/holding instrument according to one of the preceding claims, **characterized in that** the two locking elements form a circulation lock, in particular by means of a first actuation of the instrument lock (8) moving towards each other in order to latch together, and the two locking elements move towards each other again by means of a second actuation of the instrument lock (8) in order to disengage from each other again.

8. Surgical gripping/holding instrument according to one of the preceding claims, **characterized in that** the two instrument branches (2, 4) form a gap (d) between their respective intermediate regions (2c, 4c) when the instrument (1) is actuated only at the proximal actuating portions (2b, 4b) of the instrument branches (2, 4) up to a maximum possible actuating position, in particular closed position, for gripping/holding an object/tissue, and by actuating the instrument lock (8) for the first time by applying force to the two intermediate regions (2c, 4c) in such a way that they move towards each other, the gap (d) closes and the two opposite locking elements latch together, and in particular by actuating the instrument lock (8) a second time by applying force to the two intermediate regions (2c, 4c) in such a way that they move towards each other, the two locking elements are released from the latching engagement.

9. Surgical gripping/holding instrument according to one of the preceding claims, **characterized in that** the locking elements in the intermediate regions (2c, 4c) of the instrument branches (2, 4) are each arranged in such a way that they can be latched together and in particular released again by a lateral movement perpendicular to the opening/closing direction of the instrument branches (2, 4) or by a movement in the opening/closing direction of the instrument branches (2, 4).

## Revendications

1. Instrument chirurgical de préhension/maintien avec deux branches d'instrument (2, 4) accouplées de manière relativement pivotante, qui présentent respectivement une partie de préhension/maintien distale (2a, 4a), une partie d'actionnement (2b, 4b) d'instrument proximale et une zone intermédiaire (2c, 4c) reliant les parties distale et proximale de préférence en un bloc de matière, et avec un bloqueur d'instrument (8), dont les éléments de blocage pouvant être amenés en prise d'encliquetage les uns avec les autres sont disposés ou réalisés sur les branches d'instrument (2, 4), dans lequel l'actionnement de l'instrument (1) pour une ouverture et fermeture et l'actionnement de l'instrument (1) pour un blocage et déblocage de l'instrument (1) sont séparés l'un de l'autre de manière fonctionnelle par le positionnement du blocage d'instrument (8) dans la zone intermédiaire (2c, 4c) des branches d'instrument (2, 4) et peuvent donc être réalisés individuellement, **caractérisé en ce que** les zones intermédiaires (2c, 4c) des branches d'instrument (2, 4) forment les éléments d'actionnement exclusifs pour un engagement et dégagement du bloqueur d'instrument (8).

2. Instrument chirurgical de préhension/maintien selon la revendication 1, **caractérisé en ce que** les branches d'instrument (2, 4) sont en forme de barre.

3. Instrument chirurgical de préhension/maintien selon la revendication 2, **caractérisé en ce que** l'état d'encliquetage/de blocage du bloqueur d'instrument (8) ne peut être atteint que du fait que l'instrument (1) est actionné en plus pour l'actionnement de façon directe des parties d'actionnement (2b, 4b) proximales dans les zones intermédiaires (2c, 4c) se trouvant à l'extérieur des parties d'actionnement (2b, 4b), lesquelles relient les parties de préhension/maintien distales (2a, 4a) aux parties d'actionnement (2b, 4b).

4. Instrument chirurgical de préhension/maintien selon la revendication 3, **caractérisé en ce que** les deux branches d'instrument (2, 4) mobiles l'une par rapport à l'autre sont accouplées l'une à l'autre de manière pivotante à la façon d'une tenaille/pince ou à la façon d'une pincette et les parties d'actionnement (2b, 4b) peuvent respectivement de préférence être sollicitées manuellement par une force et plus préférentiellement réalisent une poignée d'instrument pour une ouverture et fermeture de l'instrument (1), dans lequel dans le cas d'une construction en tenaille/pince une articulation à charnière (6) est prévue, par l'intermédiaire de laquelle les deux branches d'instrument (2, 4) sont accouplées l'une à l'autre de manière pivotante, dans lequel le bloqueur d'instrument (8) est disposé dans la zone intermédiaire (2c, 4c) par rapport à l'articulation à charnière (6) du côté des parties d'actionnement (2b, 4b).

5. Instrument chirurgical de préhension/maintien selon l'une quelconque des revendications précédentes, **caractérisé en ce que** les branches d'instrument (2, 4) sont formées dans leurs zones intermédiaires (2c, 4c) respectives à l'extérieur des parties d'actionnement (2b, 4b) proximales pour une ouverture et fermeture de l'instrument (1), de préférence de manière arquée, de sorte que les éléments de blocage opposés du bloqueur d'instrument (8) restent hors de prise lorsque l'instrument (1) est actionné uniquement sur les parties d'actionnement (2b, 4b) proximales des branches d'instrument (2, 4) jusqu'à une position d'actionnement maximale possible, en particulier position fermée, pour une préhension/un maintien d'un objet/tissu, en particulier d'un clip pour anévrisme.

6. Instrument chirurgical de préhension/maintien selon l'une quelconque des revendications précédentes, **caractérisé en ce que** l'engagement et le dégagement du bloqueur d'instrument (8) s'effectue par déformation élastique des zones intermédiaires (2c, 4c), en particulier l'engagement et le dégagement du bloqueur d'instrument (8) s'effectue exclusivement par déformation élastique des zones intermédiaires (2c, 4c).

7. Instrument chirurgical de préhension/maintien selon l'une quelconque des revendications précédentes, **caractérisé en ce que** les deux élément de blocage forment un bloqueur rotatif, en particulier se déplacent l'un vers l'autre au moyen d'un premier actionnement du bloqueur d'instrument (8), afin de s'encliqueter l'un avec l'autre, et les deux éléments de blocage se déplacent à nouveau l'un vers l'autre au moyen d'un deuxième actionnement du bloqueur d'instrument (8), afin de se détacher à nouveau l'un de l'autre.

8. Instrument chirurgical de préhension/maintien selon l'une quelconque des revendications précédentes, **caractérisé en ce que** les deux branches d'instrument (2, 4) forment entre leurs zones intermédiaires (2c, 4c) respectives une fente (d), lorsque l'instrument (1) est actionné uniquement sur les parties d'actionnement (2b, 4b) proximales des branches d'instrument (2, 4) jusqu'à une position d'actionnement maximale possible, en particulier position fermée, pour une préhension/un maintien d'un objet/tissu, et par un premier actionnement du bloqueur d'instrument (8) par sollicitation par pression des deux zones intermédiaires (2c, 4c), de telle sorte que celles-ci se déplacent l'une vers l'autre, la fente (d) se ferme et les deux éléments de blocage opposés s'encliquètent l'un avec l'autre et en particulier par un deuxième actionnement du bloqueur d'instrument (8) par sollicitation par pression des deux zones intermédiaires (2c, 4c), de telle sorte que celles-ci se déplacent l'une vers l'autre, les deux éléments de blocage se libèrent à nouveau de la prise d'encliquetage.

9. Instrument chirurgical de préhension/maintien selon l'une quelconque des revendications précédentes, **caractérisé en ce que** les éléments de blocage dans les zones intermédiaires (2c, 4c) des branches d'instrument (2, 4) sont disposés respectivement de sorte que celles-ci peuvent s'encliqueter l'une avec l'autre par un mouvement latéral perpendiculairement à la direction d'ouverture/de fermeture des branches d'instrument (2, 4) ou par un mouvement dans la direction d'ouverture/de fermeture des branches d'instrument (2, 4) et peuvent en particulier être à nouveau libérées.
